# EUROPEAN PATENT APPLICATION

(11) **EP 4 279 573 A1**
(43) Date of publication of application: **22.11.2023**
(21) Application number: 21918838.0
(22) Date of filing: 03.06.2021
(51) Int. Cl.: C12M 1/00, C12M 1/42, C12M 1/36, C12N 15/10

(54) **DEVICE, KIT AND METHOD FOR EXTRACTING NUCLEIC ACIDS**

(30) Priority: 12.01.2021 CN 202110037413
(71) Applicant: Xiamen Zeesan Biotech Co., Ltd., Fujian 361101 (CN)
(72) Inventor: ZHAN, Wei, Xiamen, Fujian 361101 (CN); YANG, Yi, Xiamen, Fujian 361101 (CN); TANG, Yu, Xiamen, Fujian 361101 (CN); CAO, Tiantian, Xiamen, Fujian 361101 (CN)
(74) Representative: Torggler & Hofmann Patentanwälte - Innsbruck
(86) International application number: PCT/CN2021/098127
(87) International publication number: WO 2022/151640

(57) **Abstract**

The present invention provides a device, kit and method for extracting and testing nucleic acids. The devices comprises: a first container (101), a second container (102), and an air storage device (103), wherein the first container (101) is used for accommodating or containing a reagent for extracting nucleic acids; a first valve (107) is provided between the first container (101) and the second container (102); the second container (102) and the gas storage device (103) are in fluid communication; the second container (102) has a sample inlet (104) and a sample outlet (105), and the sample inlet (104) is provided with a fourth valve (109); the sample outlet device, a waste liquid pool, a collecting pool and the like. The method comprises: adding a nucleic acid sample, adsorbing nucleic acids by using magnetic beads, driving a nucleic extraction reagent, splitting, washing and eluting the nucleic acid sample, and extract nucleic acids, and significantly reduce the possibility of contamination occurring during nucleic acid extraction.

## Description

### Technical Field

The present invention relates to the field of nucleic acid extraction, in particular to a device, kit and method for extracting a nucleic acid. The device, kit and method for extracting a nucleic acid can be used for extracting a nucleic acid conveniently and efficiently, and can significantly reduce the possibility of contamination during the nucleic acid extraction process.

### Background Art

Nucleic acid is the basis of molecular biology research. Researches on gene diagnosis, gene therapy, and identification of plant varieties using genes all require extraction of high-quality nucleic acid. At present, the existing nucleic acid extraction and detection methods mainly have the following problems: (1) in the case of a huge number of biological samples, the sample processing, nucleic acid extraction and purification are prone to errors due to manual operations, and the overall operation steps are complicated, and nucleic acid extraction cannot be conducted efficiently and quickly; (2) in the most cases, molecular diagnosis needs to be carried out in the laboratory, and many grassroots units do not have the conditions to establish standard molecular diagnosis laboratories; in addition, the operators have different proficiency levels, and cross-contamination between samples is easy to occur in the process of extracting nucleic acids, and even the laboratories are also prone to contamination; (3) most of the existing nucleic acid extraction and detection instruments are relatively large in size and are not suitable for use at the sampling site, and, working in an outdoor open environment is more likely to cause sample contamination.

Therefore, there is an urgent need to develop a device for nucleic acid extraction, which is portable, can reduce the influence of human operation, and can realize fully enclosed operation, thereby reducing the possibility of contamination during nucleic acid extraction, and meeting the requirements of rapid extraction and detection in grassroots level or on-site.

### Contents of the Invention

In order to solve the above problems, the inventors of the present application have developed a device and kit for extracting a nucleic acid through a large number of experiments, which can extract nucleic acid conveniently and efficiently, and can significantly reduce the possibility of contamination during nucleic acid extraction, and reduce or even eliminate false positive test results. Based on this, the present application also provides a method for extracting a nucleic acid using the device or kit, which is simple and convenient, and can meet the requirements of rapid extraction and detection at the grassroots level or on-site.

Therefore, in one aspect, the present application provides a device for extracting a nucleic acid, which comprises: a first container 101, a second container 102 and a gas storage device 103, wherein:
the first container 101 is used to accommodate or contain a reagent for extracting a nucleic acid; and has a first valve 106;
the first container 101 and the second container 102 are in fluid communication, and a second valve 107 is arranged between them;
the second container 102 and the gas storage device 103 are in fluid communication; and
the second container 102 has a sample inlet 104 and a sample outlet 105, the sample inlet 104 is provided with a fourth valve 109; and, the sample outlet 105 is provided with a fifth valve 110.

In certain embodiments, the device is airtight. In some embodiments, the gas storage device 103 can contain or exclude gas. In some embodiments, the gas storage device 103 is variable in volume, for example, has an elastic or expandable container (e.g., an air bag), or a container with a piston (e.g., a syringe). In some embodiments, a third valve 108 is provided between the second container 102 and the gas storage device 103.

The device of the present application minimizes or avoids gas exchange with the external environment by setting a gas storage device, thereby minimizing or avoiding the possibility of contamination during the nucleic acid extraction process. Without being limited by theory, the inventors of the present application have noticed that experimental operations (e.g., vibrating, shaking, etc.) during the nucleic acid extraction can lead to the production of aerosol, and the gas exchange between the aerosol and the external environment (e.g., the experimental environment, or other nucleic acid samples extracted in parallel) can lead to contamination of the sample. Therefore, minimizing or avoiding gas exchange between the sample and the external environment can reduce or avoid the possibility of contamination during nucleic acid extraction. In some embodiments, the gas storage device 103 is an air bag, which can inhale and discharge gas through the expansion and contraction of the air bag, thereby reducing or avoiding the gas exchange between the device of the present application and the external environment. In some embodiments, the gas storage device 103 is a container with a piston, which can inhale and discharge gas through the reciprocating movement of the piston, thereby reducing or avoiding the gas exchange between the device of the present application and the external environment.

In some embodiments, the first container 101 is tubular, one end of which is provided with a first valve 106 for connecting with a driving device 111, and the other end is provided with a second valve 107 for connecting with the second container 102.

In some embodiments, the first container 101 contains a lysing solution (e.g., a cell lysing solution), a washing solution (e.g., a nucleic acid washing solution) and an eluting solution (e.g., a nucleic acid eluting solution) for nucleic acid extraction.

In certain embodiments, the lysing solution, washing solution and eluting solution are separated from each other.

In some embodiments, the lysing solution, washing solution and eluting solution are arranged in the first container 101 from the proximal end to the distal end according to the distance from the second valve 107.

In certain embodiments, the lysing solution and washing solution are separated by a first spacer. In certain embodiments, the washing solution and eluting solution are separated by a second spacer. In certain embodiments, the first and second spacers are the same or different. In some embodiments, the first spacer and the second spacer are each independently selected from the group consisting of air, mineral oil, animal oil, vegetable oil, synthetic oil, paraffin, silicone oil, and any combination thereof.

In some embodiments, the first container 101 contains a lysing solution, a first spacer, a washing solution, a second spacer and an eluting solution, and they are arranged in the first container 101 in turn from the proximal end to the distal end according to the distance from the second valve 107.

In certain embodiments, the washing solution comprises one or more portions (e.g., 1, 2, 3 or more portions), and the portions are separated from each other by an additional spacer or additional spacers. In certain embodiments, the portions have the same or different components. In certain embodiments, the additional spacers are each independently selected from the group consisting of air, mineral oil, animal oil, vegetable oil, synthetic oil, paraffin, silicone oil, and any combination thereof.

In some embodiments, a third spacer is arranged between the eluting solution and the first valve 106. In some embodiments, the third spacer is selected from the group consisting of air, mineral oil, animal oil, vegetable oil, synthetic oil, paraffin, silicone oil, and any combination thereof.

In such an embodiment, the second container may be pre-placed with a magnet (e.g., a magnetic bead) capable of adsorbing a nucleic acid. Alternatively, a magnet (e.g., a magnetic bead) capable of adsorbing a nucleic acid can also be added into the second container after the device is started.

Alternatively, in some embodiments, the first container 101 contains a washing solution (e.g., a nucleic acid washing solution) and an eluting solution (e.g., a nucleic acid eluting solution) for nucleic acid extraction.

In certain embodiments, the washing solution and eluting solution are separate from each other.

In some embodiments, the washing solution and the eluting solution are arranged in the first container 101 from the proximal end to the distal end according to the distance from the second valve 107.

In certain embodiments, the washing solution and eluting solution are separated by a first spacer.

In some embodiments, the first container 101 contains a washing solution, a first spacer and an eluting solution, and they are arranged in turn in the first container 101 from the proximal end to the distal end according to the distance from the second valve 107.

In certain embodiments, the wash solution comprises one or more portions (e.g., 1, 2, 3 or more portions), and the portions are separated from each other by an additional spacer or additional spacers. In certain embodiments, the portions have the same or different components. In certain embodiments, the additional spacers are each independently selected from the group consisting of air, mineral oil, animal oil, vegetable oil, synthetic oil, paraffin, silicone oil, and any combination thereof.

In some embodiments, a second spacer is arranged between the eluting solution and the first valve 106. In certain embodiments, the first and second spacers are the same or different. In some embodiments, the first spacer and the second spacer are each independently selected from the group consisting of air, mineral oil, animal oil, vegetable oil, synthetic oil, paraffin, silicone oil, and any combination thereof.

In some embodiments, the second container may be pre-placed with a magnet (e.g., a magnetic bead) capable of adsorbing a nucleic acid. Alternatively, a magnet (e.g., a magnetic bead) capable of adsorbing a nucleic acid can also be added into the second container after the device is started. In addition, in some embodiments, the second container may be pre-placed with a lysing solution (e.g., a cell lysing solution) for nucleic acid extraction. Alternatively, the lysing solution (e.g., a cell lysing solution) for nucleic acid extraction can also be added to the second container after the device is started. Therefore, in some embodiments, the second container 102 contains a lysing solution (e.g., a cell lysing solution) for extracting a nucleic acid, and/or a magnet (e.g., a magnetic bead) capable of adsorbing a nucleic acid.

In certain embodiments, one or more spacers, for example, 1, 2, 3 or more spacers, may be used between each two reagents. The one or more spacers are each independently selected from the group consisting of air, mineral oil, animal oil, vegetable oil, synthetic oil, paraffin, silicone oil, and any combination thereof.

As used herein, the spacer used in the present application may be liquid, gaseous, or solid, and it is immiscible with the reagent used for nucleic acid extraction. In some embodiments, the spacer used in the present application is stable, and even under conditions such as vibration, it can space the reagents at both ends of the spacer so that the two reagents do not contact each other. In addition, it can be understood that any substance that can realize this function can be used as the spacer in the present application. Therefore, the spacer used in the present application is not limited to the substances listed above.

In some embodiments, the first container 101 has a first chamber, a second chamber, and a third chamber separated from each other; wherein, the first chamber is used to accommodate or contain a lysing solution for nucleic acid extraction (e.g., a cell lysing solution); the second chamber is used to accommodate or contain a washing solution for nucleic acid extraction (e.g., a nucleic acid washing solution); the third chamber is used to accommodate or contain an eluting solution for nucleic acid extraction (e.g., a nucleic acid eluting solution); and, the second valve 107 can control the type of reagent flowing into the second container 102. In some embodiments, the second container may be pre-placed with a magnet (e.g., a magnetic bead) capable of adsorbing a nucleic acid. Alternatively, a magnet (e.g., a magnetic bead) capable of adsorbing a nucleic acid can also be added into the second container after the device is started.

In some embodiments, the first container 101 has a first chamber and a second chamber separated from each other; wherein, the first chamber is used to accommodate or contain a washing solution (e.g., a nucleic acid washing solution) for nucleic acid extraction; the second chamber is used to accommodate or contain an eluting solution (e.g., a nucleic acid eluting solution) for nucleic acid extraction; and, the second valve 107 can control the type of reagent flowing into the second container 102. In some embodiments, the second container may be pre-placed with a magnet (e.g., a magnetic bead) capable of adsorbing a nucleic acid. Alternatively, a magnet (e.g., a magnetic bead) capable of adsorbing a nucleic acid can also be added into the second container after the device is started. In addition, in some embodiments, the second container 102 may be pre-placed with a lysing solution (e.g., a cell lysing solution) for nucleic acid extraction. Alternatively, the lysing solution (e.g., a cell lysing solution) for nucleic acid extraction can also be added to the second container after the device is started. Therefore, in some embodiments, the second container 102 contains a lysing solution (e.g., a cell lysing solution) for nucleic acid extraction, and/or a magnet (e.g., a magnetic bead) capable of adsorbing a nucleic acid.

In some embodiments, the first valve 106 is used to control fluid communication between the first container 101 and the driving device 111. In some embodiments, the device also has a driving device 111 in fluid communication with the first container 101 through the first valve 106. In some embodiments, the driving device 111 is detachable; and in some embodiments, the driving device 111 is a driving pump.

In some embodiments, the second container 102 contains a magnet (e.g., a magnetic bead) capable of adsorbing a nucleic acid, and/or a lysing solution (e.g., a cell lysing solution) for nucleic acid extraction. In some embodiments, the magnetic bead is one or more selected from the group consisting of the follows: silica gel plasma membrane magnetic bead, amino magnetic bead, hydroxyl magnetic bead, formyl magnetic bead, and cellulose-coated magnetic bead.

In some embodiments, the sample outlet 105 is connected via a fifth valve 110 to a waste liquid pool 112, or a collection pool 113, or a waste liquid pool 112 and a collection pool 113. In some embodiments, the fifth valve 110 can control the flow direction of the fluid that passes through the fifth valve 110. In some embodiments, the collection pool 113 contains a reagent for detecting a nucleic acid. In some embodiments, the waste liquid pool 112 and/or the collection pool 113 are detachable.

In certain embodiments, the nucleic acid is selected from the group consisting of DNA, RNA, or any combination thereof. The device of the present application can be used to extract DNA, RNA or any combination thereof.

In a second aspect, the present application provides a kit, which comprises the aforementioned device. In some embodiments, the kit further comprises one or more parts or components selected from the group consisting of: a reagent for nucleic acid extraction, a spacer, a magnet (e.g., a magnetic bead) capable of adsorbing a nucleic acid, a driving device, a waste liquid pool, and a collection pool.

In certain embodiments, the kit further comprises a reagent for nucleic acid extraction. In some embodiments, the reagent for nucleic acid extraction is selected from the group consisting of a lysing solution (e.g., a cell lysing solution), a washing solution (e.g., a nucleic acid washing solution), an eluting solution (e.g., a nucleic acid eluting solution), or any combination thereof. In certain embodiments, the lysing solution, washing solution and eluting solution are separated from each other.

In certain embodiments, the kit further comprises one or more spacers. In certain embodiments, the one or more spacers are each independently selected from the group consisting of air, mineral oil, animal oil, vegetable oil, synthetic oil, paraffin, silicone oil, and any combination thereof.

In certain embodiments, the kit further comprises a magnet (e.g., a magnetic bead) capable of adsorbing a nucleic acid. In some embodiments, the magnetic bead is one or more selected from the group consisting of the followings: silica gel plasma membrane magnetic bead, amino magnetic bead, hydroxyl magnetic bead, formyl magnetic bead, and cellulose-coated magnetic bead.

In certain embodiments, the kit further comprises a driving device. In some embodiments, the driving device is a drive pump. In some embodiments, the driving device can be in fluid communication with the first container 101 through the first valve 106.

In certain embodiments, the kit further comprises a waste liquid pool. In some embodiments, the waste liquid pool can be in fluid communication with the sample outlet 105 through the fifth valve 110.

In certain embodiments, the kit further comprises a collection pool. In some embodiments, the collection pool can be in fluid communication with the sample outlet 105 through the fifth valve 110. In certain embodiments, the collection pool contains a reagent for nucleic acid detection.

In a third aspect, the present application provides a method for nucleic acid extraction, the method comprising, using the device of the present application or the kit of the present application to extract a nucleic acid from a sample.

In some embodiments, the method comprises:
(1) providing a sample containing a nucleic acid, and the aforementioned device or the aforementioned kit;
(2) loading the sample into the second container 102 through the sample inlet 104, and closing the fourth valve 109; wherein, the second container 102 contains a magnet (e.g., a magnetic bead) capable of adsorbing the nucleic acid;
(3) contacting, mixing and incubating a lysing solution, a magnet (e.g., magnetic bead) capable of adsorbing a nucleic acid with the sample in the second container 102 for an appropriate time;
(4) after incubation, opening the third valve 108 and the fifth valve 110, discharging all the solution in the second container 102 to the waste liquid pool 112 through the fifth valve 110, and retaining the magnet;
(5) closing the fifth valve 110, driving the washing solution that is preloaded in the first container 101 into the second container 102 by using the driving device 111, contacting, mixing and incubating with the magnet for an appropriate time; wherein, during the driving process, the first valve 106, the second valve 107 and the third valve 108 are in an open state, and the fourth valve 109 and the fifth valve 110 are in a closed state; after the driving process is completed, the first valve 106 and the second valve 107 are closed, and optionally the third valve 108 is closed;
(6) after incubation, opening the third valve 108 and the fifth valve 110, discharging all the solution in the second container 102 to the waste liquid pool 112 through the fifth valve 110, and retaining the magnet;
   optionally, repeating steps (5) and (6) one or more times;
(7) closing the fifth valve 110, driving the eluting solution that is preloaded in the first container 101 into the second container 102 by using the driving device 111, contacting, mixing and incubating with the magnet for an appropriate time; wherein, during the driving process, the first valve 106, the second valve 107 and the third valve 108 are in an open state, and the fourth valve 109 and the fifth valve 110 are in a closed state; after the driving process is completed, the first valve 106 and the second valve 107 are closed, and optionally the third valve 108 is closed;
(8) after the incubation, the third valve 108 and the fifth valve 110 are opened, and all the solution in the second container 102 is collected into the collection pool 113 through the fifth valve 110, thereby obtaining a solution containing the nucleic acid to be extracted.

In some embodiments, the magnet used in step (2) has been preloaded in the second container 102. In some embodiments, the magnet used in step (2) is loaded into the second container 102 together with the sample. In some embodiments, the magnet used in step (2) is loaded into the second container 102 after the sample is loaded.

In some embodiments, the lysing solution used in step (3) has been preloaded in the second container 102. In some embodiments, the lysing solution used in step (3) is loaded into the second container 102 together with the sample. In some embodiments, the lysing solution used in step (3) is loaded into the second container 102 after the sample is loaded. In some embodiments, the lysing solution used in step (3) has been preloaded in the first container 101, and is driven into the second container 102 by the driving device 111, wherein, during the driving process, the first valve 106, the second valve 107 and the third valve 108 are in an open state, and the fourth valve 109 and the fifth valve 110 are in a closed state; after the driving process is completed, the first valve 106 and the second valve 107 are closed, and optionally the third valve 108 is closed.

In certain embodiments, the nucleic acid adsorbed to the magnet can be washed more than one times. Therefore, steps (5) and (6) can be repeated one or more times. In some embodiments, the first container 101 is tubular and contains one or more sections of washing solution, whereby the nucleic acid adsorbed on the magnet can be washed one or more times.

In some embodiments, after step (8), the extracted nucleic acid is detected. In some embodiments, the collection pool 113 contains a reagent for nucleic acid detection. In some embodiments, the extracted nucleic acid is detected in the collection pool 113.

In the fourth aspect, the present application provides a use of the aforementioned device or the aforementioned kit for nucleic acid extraction.

### Definition of terms

As used herein, the term "fluid" refers to a flowable substance that deforms under the action of a shear force. For example, the most common fluids are gases and liquids. As used herein, the term "fluid communication" is used to refer to a connected relationship between two components in a device that enables fluid (e.g., gases and liquids) to communicate between the two components.

As used herein, the term "reagent for nucleic acid extraction" refers to a reagent used in a nucleic acid extraction process. The reagent for nucleic acid extraction generally comprises lysing solution, nucleic acid washing solution, and nucleic acid eluting solution.

As used herein, the term "lysing solution" refers to a solution in which a nucleic acid in a sample is freed. Such lysing solutions are well known to those skilled in the art and are conveniently formulated or commercially available. In certain embodiments, the lysing solution is capable of lysing a cell to release a nucleic acid (e.g., genomic DNA, plasmid DNA, mitochondrial DNA, chloroplast DNA, total RNA, mRNA, tRNA, miRNA, etc.) contained in the cell, and dissolving/freeing it in the lysing solution. In such embodiments, the lysing solution is also referred to as a cell lysing solution.

As used herein, the term "nucleic acid washing solution" refers to a solution used to remove an impurity (e.g., cell debris, protein, polysaccharide, plasma membrane, etc.) from a nucleic acid-containing system during nucleic acid extraction. Such nucleic acid washing solutions are well known to those skilled in the art, and are conveniently formulated or commercially available. Herein, "nucleic acid washing solution" and "washing solution" are used interchangeably.

As used herein, the term "nucleic acid eluting solution" refers to a solution for separating nucleic acid from a nucleic acid-containing system during nucleic acid extraction, which can dissolve the nucleic acid and store the nucleic acid stably. Such nucleic acid eluting solutions are well known to those skilled in the art, and are conveniently formulated or commercially available. Herein, "nucleic acid eluting solution" and "eluting solution" are used interchangeably.

Those skilled in the art will understand that, according to factors such as the type of sample (e.g., cell culture, isolated tissue, body fluid, etc.) from which nucleic acid is to be extracted, the type of nucleic acid to be extracted (e.g., DNA, such as genomic DNA, plasmid DNA, mitochondrial DNA, chloroplast DNA, etc.; or RNA, such as total RNA, mRNA, tRNA, miRNA, etc.), and the extraction method used, the components of the lysing solution (e.g., cell lysing solution), nucleic acid washing solution and nucleic acid eluting solution are adjustable. And, such adjustments are well within the capabilities of those skilled in the art.

For example, when the nucleic acid to be extracted is plasmid DNA in cell culture, the lysing solution may usually contain a cell-lysing reagent (e.g., SDS, Triton X-100, NP-40, guanidine isothiocyanate (GITC), etc.) and a salt (e.g., Tris, EDTA, NaCl, etc.). When the nucleic acid to be extracted is genomic DNA, the lysing solution usually also contains cetyltrimethylammonium bromide (CTAB). For example, the commonly used cell lysing solution formula for DNA extraction can be 4M guanidine hydrochloride, 50mM Tris-HCl, 10mM EDTA, 15% Triton X100, pH 6.5. Commonly used washing solution for washing DNA may comprise salt (e.g., Tris, EDTA, NaCl, etc.) and ethanol, etc. For example, the commonly used washing solution formula for washing DNA can be 100 mM NaCl, 50 mM Tris-HCl (pH 7.8), 75% absolute ethanol. The commonly used eluting solution for eluting DNA can be TE solution or sterile water, wherein the TE solution is usually prepared from Tris and EDTA. For example, a commonly used eluting solution formula for eluting DNA can be 10 mM Tris-HCl, 1 mM EDTA, pH 8.5.

For example, when the nucleic acid to be extracted is total RNA in cells, the lysing solution usually comprises a cell-lysing reagent (e.g., Trizol, etc.), a RNase inhibitor (e.g., 8-hydroxyquinoline, β-mercaptoethanol, DEPC, etc.), a protein denaturant (e.g., GIT, GuHCl, etc.). Commonly used washing solution for washing RNA may comprise a RNase inhibitor (e.g., 8-hydroxyquinoline, β-mercaptoethanol, DEPC, etc.) and ethanol. The commonly used eluting solution for eluting RNA can be RNase-free sterile water.

It is easy to understand that the lysing solution, nucleic acid washing solution and nucleic acid eluting solution involved in the present invention are not limited to the formulations and components listed above, and are not limited to the formulations and components used in the examples, and as mentioned above, they can be adjusted and changed according to actual needs.

As used herein, the term "magnet capable of adsorbing nucleic acid" refers to a magnetic substance with an improved and modified surface (e.g., magnetic particle), which can specifically recognize and bind a nucleic acid molecule on a microscopic interface. In certain embodiments, the magnetic substance is a paramagnetic substance (e.g., paramagnetic particle), and preferably a superparamagnetic substance (e.g., superparamagnetic particle). The magnet with paramagnetism is particularly advantageous because they can gather quickly in a magnetic field and then scatter evenly after leaving the magnetic field. The method of adsorbing/extracting nucleic acid using magnetic substance (also referred to as "magnetic bead method") is well known in the art, and a variety of magnetic beads for nucleic acid extraction have been developed, including, for example, magnetic bead for DNA extraction and magnetic bead for RNA extraction, such as silica gel plasma membrane magnetic bead, amino magnetic bead, hydroxyl magnetic bead, formyl magnetic bead, cellulose-coated magnetic bead, etc. Such magnetic beads are commercially available. In some cases, it is particularly advantageous to use the magnetic bead method for nucleic acid extraction because it can be easily automated. Thus, in certain preferred embodiments, the device and kit of the present invention can be used to extract nucleic acid by magnetic bead method.

As used herein, the term "nucleic acid" comprises single- and double-stranded deoxyribonucleic acid (DNA) and ribonucleic acid (RNA). In the present application, the nucleic acid comprises, but is not limited to, DNA, such as plasmid DNA, genomic DNA, mitochondrial DNA, chloroplast DNA, etc.; and RNA, such as total RNA, mRNA, tRNA, miRNA, etc.

### Beneficial Effects of the Invention

The device, kit and method for nucleic acid extraction provided in the present application can realize the extraction and detection of a nucleic acid from a sample in a sealed state, and greatly reduce or avoid the gas exchange between the device and the external environment, thus significantly reducing the possibility of environmental contamination and cross-contamination between samples during the nucleic acid extraction process, reducing or even eliminating false positive test results. In addition, the nucleic acid extraction device of the present application has a simple structure, is easy to carry, and the extraction method is easy to operate, and can meet the requirements of rapid extraction and detection at the grassroots level or on site.

### Brief Description of the Drawings

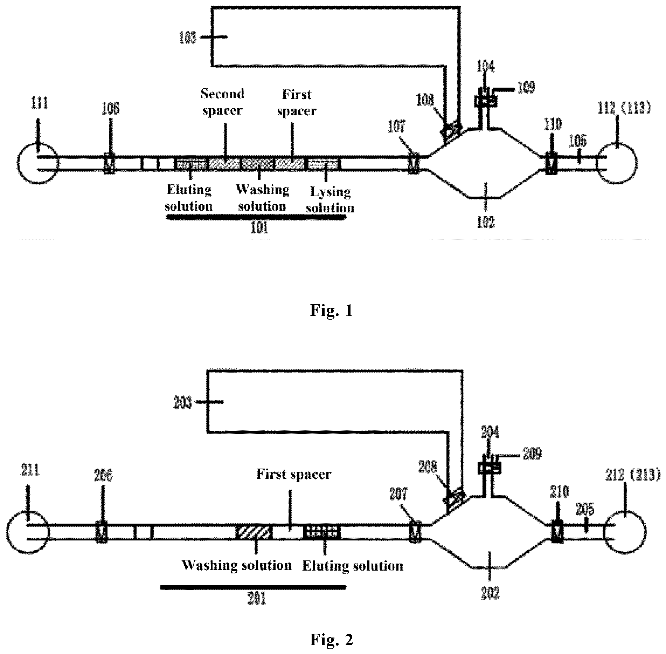

Fig. 1 shows a device for nucleic acid extraction designed by the present application, which comprises: a first container 101, a second container 102 and a gas storage device 103, wherein:
   the first container 101 contains a lysing solution, a washing solution and an eluting solution separated from each other by a spacer; and is connected to a driving device 111 via a first valve 106;
   the first container 101 and the second container 102 are in fluid communication, and a second valve 107 is arranged between them;
   the second container 102 and the gas storage device 103 are in fluid communication, and a third valve 108 is arranged between them;
   the second container 102 has a sample inlet 104 and a sample outlet 105, and the sample inlet 104 is provided with a fourth valve 109; a magnetic bead can be contained in the second container;
   the sample outlet 105 is provided with a fifth valve 110; and, the sample outlet 105 is connected to a waste liquid pool 112 or a collection pool 113 via the fifth valve 110.
Fig. 2 shows a device for nucleic acid extraction designed by the present application, which comprises: a first container 201, a second container 202 and a gas storage device 203, wherein:
   the first container 201 contains a washing solution and an eluting solution separated from each other by a spacer; and is connected to a driving device 211 via a first valve 206;
   the first container 201 and the second container 202 are in fluid communication, and a second valve 207 is arranged between them;
   the second container 202 and the gas storage device 203 are in fluid communication, and a third valve 208 is arranged between them;
   the second container 202 has a sample inlet 204 and a sample outlet 205, and the sample inlet 204 is provided with a fourth valve 209; a lysing solution and/or a magnetic bead can be contained in the second container;
   the sample outlet 205 is provided with a fifth valve 210; and, the sample outlet 205 is connected to a waste liquid pool 212 or a collection pool 213 via the fifth valve 210.

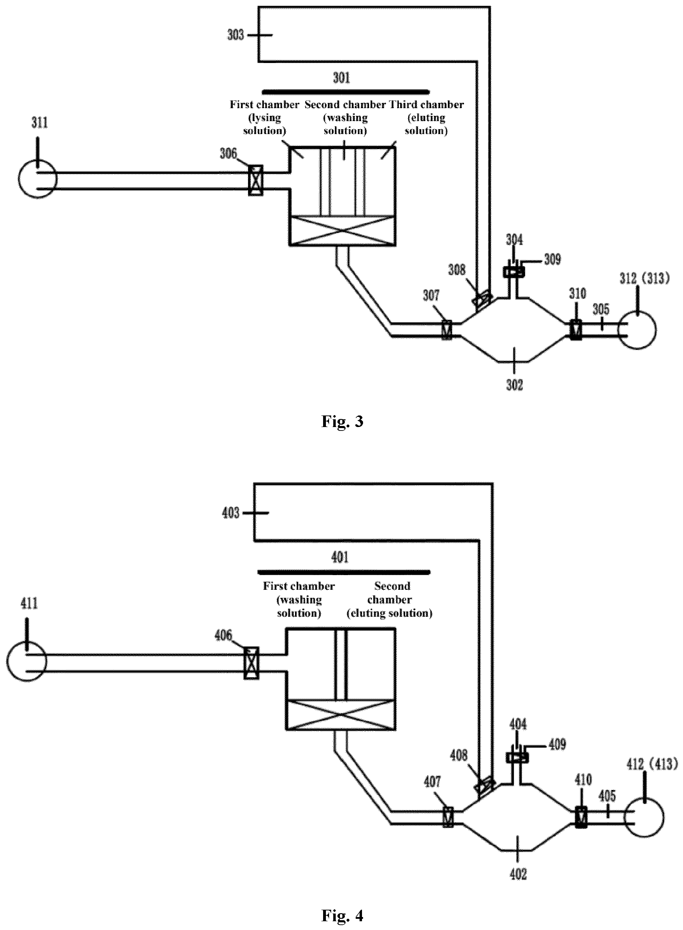

Fig. 3 shows a device for nucleic acid extraction designed by the present application, which comprises: a first container 301, a second container 302 and a gas storage device 303, wherein:
   the first container 301 has a first chamber, a second chamber and a third chamber separated from each other; wherein, the first chamber contains a lysing solution, the second chamber contains a washing solution, and the third chamber contains an eluting solution;
   the first container is connected to a driving device 311 via a first valve 306;
   the first container 301 and the second container 302 are in fluid communication, and a second valve 307 is arranged between them; the second valve 307 can control the type of fluid flowing into the second container 302;
   the second container 302 and the gas storage device 303 are in fluid communication, and a third valve 308 is arranged between them;
   the second container 302 has a sample inlet 304 and a sample outlet 305, and the sample inlet 304 is provided with a fourth valve 309; a magnetic bead can be contained in the second container;
   the sample outlet 305 is provided with a fifth valve 310; and, the sample outlet 305 is connected to a waste liquid pool 312 or a collection pool 313 via the fifth valve 310.
Fig. 4 shows a device for nucleic acid extraction designed by the present application, which comprises: a first container 401, a second container 402 and a gas storage device 403, wherein:
   the first container 401 has a first chamber and a second chamber separated from each other; wherein, the first chamber contains a washing solution, and the second chamber contains an eluting solution;
   the first container is connected to a driving device 411 via a first valve 406;
   the first container 401 and the second container 402 are in fluid communication, and a second valve 407 is arranged between them; the second valve 407 can control the type of fluid flowing into the second container 402;
   the second container 402 and the gas storage device 403 are in fluid communication, and a third valve 408 is arranged between them;
   the second container 402 has a sample inlet 404 and a sample outlet 405, and the sample inlet 404 is provided with a fourth valve 409; a magnetic bead and/or a lysing solution can be contained in the second container;
   the sample outlet 405 is provided with a fifth valve 410; and, the sample outlet 405 is connected to a waste liquid pool 412 or a collection pool 413 via the fifth valve 410.

### Specific Models for Carrying Out the Invention

Embodiments of the present invention will be described in detail below in conjunction with examples, but those skilled in the art will understand that the following examples are only for illustrating the present invention and should not be considered as limiting the scope of the present invention. Those without indicating the specific conditions in the examples were carried out according to the conventional conditions or the conditions suggested by the manufacturer. The reagents or instruments without indicating manufacturer were all commercially available conventional products.

### Example 1: Configuration and use method of device

Fig. 1 showed a device for nucleic acid extraction designed by the present application, which comprised: a first container **101,** a second container **102** and a gas storage device **103,** wherein: the first container **101** contained a lysing solution, a first spacer, a washing solution, a second spacer and an eluting solution in sequence from the proximal end to the distal end according to the distance from a second valve **107;** and, was connected to a driving device **111** via a first valve **106;** the first container **101** was in fluid communication with the second container **102,** and the second valve **107** was arranged between them; the second container **102** and the gas storage device **103** were in fluid communication, and a third valve **108** was arranged between them; the second container **102** had a sample inlet **104** and a sample outlet **105,** and the sample inlet **104** was provided with a fourth valve **109;** a magnetic bead could be contained in the second container; the sample outlet **105** was provided with a fifth valve **110;** and, the sample outlet **105** was connected to a waste liquid pool **112** or a collection pool **113** via the fifth valve **110.**

When the device was used to extract a nucleic acid from a sample, first, the sample containing the nucleic acid was loaded into the second container **102** containing a magnet (e.g., a magnetic bead) capable of adsorbing the nucleic acid through the sample inlet **104,** and the fourth valve **109** was closed. Then, the lysing solution in the first container **101** was driven into the second container **102** by the driving device **111,** wherein, in the driving process, the first valve **106,** the second valve **107** and the third valve **108** were in an open state, and the fourth valve **109** and fifth valve **110** were in a closed state. During this period, the gas in the second container **102** would enter the gas storage device **103** through the third valve **108** instead of entering the external environment. After the driving process was completed, the first valve **106** and the second valve **107** were closed, and the third valve **108** was optionally closed, and the lysing solution, the magnet and the sample were mixed in the second container **102** and incubated for a suitable time. After incubation, the third valve **108** and the fifth valve **110** were opened, and all the solution in the second container **102** was discharged through the fifth valve **110** to the waste liquid pool **112,** and the magnet was retained. During this period, the gas in the gas storage device **103** would enter the second container **102** through the third valve **108** to prevent the entry of gas from the external environment.

Subsequently, the fifth valve **110** was closed, the driving device **111** was used to drive the washing solution in the first container **101** into the second container **102,** which was allowed to contact, mix and incubate with the magnet for a suitable time; wherein, during the driving process, the first valve **106,** the second valve **107** and the third valve **108** were in an open state, and the fourth valve **109** and the fifth valve **110** were in a closed state. During this period, the gas in the second container **102** would enter the gas storage device **103** through the third valve **108** instead of entering the external environment. After the driving process was completed, the first valve **106** and the second valve **107** were closed, and the third valve **108** was optionally closed, and the washing solution, the magnet and the sample were mixed in the second container **102** and incubated for a suitable time. After the incubation, the third valve **108** and the fifth valve **110** were opened, and all the solution in the second container **102** was drained through the fifth valve **110** to the waste liquid pool **112,** and the magnet was retained. During this period, the gas in the gas storage device **103** would enter the second container **102** through the third valve **108** to prevent the entry of gas from the external environment. Optionally, the washing process could be repeated one or more times.

Subsequently, the fifth valve **110** was closed, the driving device **111** was used to drive the eluting solution in the first container **101** into the second container **102,** which was allowed to contact, mix and incubate with the magnet for a suitable time; wherein, during the driving process, the first valve **106,** the second valve **107** and the third valve **108** were in the open state, and the fourth valve **109** and the fifth valve **110** were in the closed state. During this period, the gas in the second container **102** would enter the gas storage device **103** through the third valve **108** instead of entering the external environment. After the driving process was completed, the first valve **106** and the second valve **107** were closed, and the third valve **108** was optionally closed, and the eluting solution, the magnet and the sample were mixed in the second container **102** and incubated for a suitable time. After the incubation, the third valve **108** and the fifth valve **110** were opened, and all the solution in the second container **102** was discharged to the collection pool **113** through the fifth valve **110,** thereby obtaining a solution containing the nucleic acid to be extracted. During this period, the gas in the gas storage device **103** would enter the second container **102** through the third valve **108** to prevent the entry of gas from the external environment.

In this device, the storage device **103** was used to buffer air, and gas could be inhaled and expelled. The existence of the storage device enabled the gas in the entire device to circulate repeatedly. Therefore, the whole device did not exchange gas with the outside, which enabled the sealing of the device, and could avoid the contamination of the sample caused by the environment during the nucleic acid extraction process. The second container **102** was used for lysing, binding, washing and eluting during the nucleic acid extraction process. The sample outlet **105** was used to discharge a waste liquid generated during the nucleic acid extraction process (connected to the waste liquid pool **112**) or to collect an extracted nucleic acid (connected to the collection pool **113**). The waste liquid pool **112** and the collection pool **113** could be detachable, or the flow direction of fluid (waste liquid and nucleic acid solution) could be controlled by the fifth valve **110**.

Fig. 2 showed another device for nucleic acid extraction designed by the present application, which comprised: a first container **201,** a second container **202** and a gas storage device **203,** wherein: the first container **201** contained a washing solution, a first spacer and an eluting solution from the proximal end to the distal end according to the distance from a second valve **207;** and, was connected to a driving device **211** via a first valve **206;** the first container **201** and the second container **202** were in fluid communication, and a second valve **207** was provided between them; the second container **202** and the gas storage device **203** were in fluid communication, and a third valve **208** was provided between them; the second container **202** had a sample inlet **204** and a sample outlet **205,** and the sample inlet **204** was provided with a fourth valve **209;** the second container could contain a lysing solution and/or a magnetic bead; the sample outlet **205** was provided with a fifth valve **210;** and, the sample outlet **205** was connected to a waste liquid pool **212** or a collection pool **213** via the fifth valve **210.**

The device shown in Fig. 2 differed from the device shown in Fig. 1 mainly in that the reagents preloaded in the first container were different. In the device of Fig. 2, the first container **201** was only loaded with the washing solution, the first spacer and the eluting solution, but did not contain the lysing solution. Therefore, during the use of the device, the driving device **211** was no longer used to drive the lysing solution into the second container **202,** but the lysing solution was preloaded in the second container **202,** or the lysing solution was added into the second container **202** through the sample inlet **204.** Other than that, the method of using the device shown in Fig. 2 was basically the same as that of using the device shown in Fig. 1.

Fig. 3 showed another device for nucleic acid extraction designed by the present application, which comprised: a first container **301,** a second container **302** and a gas storage device **303,** wherein: the first container **301** had a first chamber, a second chamber and a third chamber which were spaced apart from each other; wherein, the first chamber contained the lysing solution, the second chamber contained the washing solution, and the third chamber contained the eluting solution; the first container was connected with a driving device **311** through a first valve **306;** the first container **301** and the second container **302** were in fluid communication, and a second valve **307** was arranged between them, and the second valve **307** could control the type of fluid flowing into the second container **302;** the second container **302** and the gas storage device **303** were in fluid communication, and a third valve **308** was arranged between them; the second container **302** had a sample inlet **304** and a sample outlet **305,** the sample inlet **304** was provided with a fourth valve **309;** the second container could contain a magnetic bead; the sample outlet **305** was provided with a fifth valve **310;** and, the sample outlet **305** was connected to the waste liquid pool **312** or the collection pool **313** through the fifth valve **310.**

The device shown in Fig. 3 differed from the device shown in Fig. 1 mainly in that the first container was designed differently. In the device of Fig. 3, the first container **301** separated the lysing solution, the washing solution and the eluting solution through different chambers (rather than spacers), and used the second valve **307** (rather than the order of the reagents) to control the type of reagent flowing into the second container **302.** Therefore, during the use of the device, the position of the second valve **307** should be adjusted according to specific steps, so as to control the type of reagent flowing into the second container **302.** Other than that, the method of using the device shown in Fig. 3 was basically the same as that of using the device shown in Fig. 1.

Fig. 4 showed a device for nucleic acid extraction designed by the present application, which comprised: a first container **401,** a second container **402** and a gas storage device **403,** wherein: the first container **401** had a first chamber and a second chamber that were spaced between each other; wherein, the first chamber contained a washing solution, and the second chamber contained an eluting solution; the first container was connected with the driving device **411** via a first valve **406;** the first container **401** and the second container **402** were in fluid communication, and a second valve **407** was arranged between them, and the second valve **407** could control the type of fluid flowing into the second container **402;** the second container **402** and the gas storage device **403** were in fluid communication, and a third valve **408** was provided between them; the second container **402** had a sample inlet **404** and a sample outlet **405,** and the sample inlet **404** was provided with a fourth valve **409;** the second container could contain a magnetic bead and/or a lysing solution; the sample outlet **405** was provided with a fifth valve **410;** and the sample outlet **405** was connected to a waste liquid pool **412** or a collection pool **413** via the fifth valve **410.**

The device shown in Fig. 4 differed from the device shown in Fig. 3 mainly in that the reagents preloaded in the first container were different. In the device of Fig. 4, the first container **401** was only loaded with the washing solution, the first spacer and the eluting solution, but did not contain the lysing solution. Therefore, during the use of the device, the driving device **411** was no longer used to drive the lysing solution into the second container **402,** but the lysing solution was preloaded in the second container **402,** or the lysing solution was added into the second container **402** through the sample inlet **404.** Other than that, the method of using the device shown in Fig. 4 was basically the same as that of using the device shown in Fig. 3.

The driving devices, the waste liquid pools and the collection pools used in Fig. 1 to Fig. 4 could be comprised in the devices, or they could be detachable and provided as independent supporting parts.

### Example 2: Extraction and detection of nucleic acid

In this example, nucleic acid was extracted using the device designed in Fig. 1, wherein the first container was a tubular container with an inner wall diameter of 1 mm. In the first container, 50 µL of eluting solution (the formula was: 10 mM Tris-HCl, 1 mM EDTA, pH 8.5), 3 ml of washing solution (the formula was: 100 mM NaCl, 50 mM Tris-HCl (pH 7.8), 75% absolute ethanol), 2ml of lysing solution (the formula was: 4M guanidine hydrochloride, 50mM Tris-HCl, 10mM EDTA, 15% Triton X100, pH 6.5) was placed. Both the first spacer and the second spacer were air. The second container was preloaded with silica gel plasma membrane magnetic bead (containing ferric oxide and silicon dioxide) for nucleic acid extraction. The storage device was an air bag. The driving device was a driving pump. Before the kit was used, all the valves were closed to prevent the reagents in the first container from flowing out, and to prevent the gas from the external environment from entering the device.

The above device was used to extract nucleic acid, and the specific steps were as follows:
(1) The fourth valve was opened and 200 µL of a nucleic acid sample was added thereto; then, the fourth valve was closed.
(2) The first valve, the second valve and the third valve were opened, and the lysing solution in the first container was driven to flow into the second container by the driving pump. Then, the first valve, the second valve and the third valve were closed.
(3) The sample, a magnetic bead and the lysing solution were fully mixed in the second container by using a shaker or a magnetic stirrer; incubated at room temperature for 2-5 minutes to complete the lysis of the sample and the binding of the nucleic acid to the magnetic bead. After the binding was completed, the shaker or the magnetic stirrer was turned off.
(4) A magnet was used to adsorb the magnetic bead in the second container to the inner wall of the second container.
(5) The third valve and the fifth valve were opened, and the waste lysing solution in the second container was discharged into the waste liquid pool through the sample outlet. Then, the fifth valve was closed.
(6) The first valve, the second valve and the third valve were opened, and the washing solution in the first container was driven to flow into the second container by the driving pump. Then, the first valve, the second valve and the third valve were closed.
(7) The sample, the magnetic bead and the washing solution in the second container were fully mixed by using a shaker or a magnetic stirrer; incubated at room temperature for 1-2 minutes to complete the washing of the nucleic acid bound to the magnetic bead. After the washing was completed, the shaker or the magnetic stirrer was turned off.
(8) A magnet was used to adsorb the magnetic bead in the second container to the inner wall of the second container.
(9) The third valve and the fifth valve were opened, and the waste washing solution in the second container was discharged into the waste liquid pool through the sample outlet. Then, the fifth valve was closed.
(10) The first valve, the second valve and the third valve were opened, and the eluting solution in the first container was driven to flow into the second container by the driving pump. Then, the first valve, the second valve and the third valve were closed.
(11) The sample, the magnetic bead and the eluting solution in the second container were fully mixed by using a shaker or a magnetic stirrer; incubated at room temperature for 2-5 minutes to elute the nucleic acid bound to the magnetic bead. After the elution was completed, the shaker or the magnetic stirrer was turned off.
(12) A magnet was used to adsorb the magnetic bead in the second container to the inner wall of the second container.
(13) The third valve and the fifth valve were opened, and the nucleic acid eluting solution in the second container was discharged into the collection pool through the sample outlet, the collection pool contained a pre-loaded freeze-dried powder reagent (comprising: 50 µmol of primer, 50 µmol of probe, 5U of Taq DNA polymerase, 10× PCR buffer, 100 mmol of Mg²⁺, 10 mmol of dNTP) for nucleic acid detection. Then, the third and fifth valves were closed.
(14) The nucleic acid in the collection pool was automatically detected through a supporting equipment. Wherein, the supporting instrument was an instrument capable of performing PCR amplification and real-time fluorescence detection. In the present example, a real-time fluorescent PCR instrument was used, wherein a thermal cycle module provided different reaction temperatures for the collection pool according to the needs of PCR amplification, and a fluorescence signal detection module detected a fluorescence signal of the PCR amplification reactants at a specific stage of the reaction.

Although the specific models for carrying out the present invention have been described in detail, those skilled in the art will understand that: according to all the teachings that have been disclosed, various modifications and changes can be made to the details, and these changes are all within the protection scope of the present invention. The full scope of the present invention is given by the appended claims and any equivalents thereof.

## Claims

1. A device for nucleic acid extraction, comprising: a first container (101), a second container (102) and a gas storage device (103), wherein:
the first container (101) is used to accommodate or contain a reagent for nucleic acid extraction; and has a first valve (106);
the first container (101) and the second container (102) are in fluid communication, and a second valve (107) is arranged between them;
the second container (102) and the gas storage device (103) are in fluid communication; and
the second container (102) has a sample inlet (104) and a sample outlet (105), and the sample inlet (104) is provided with a fourth valve (109); and, the sample outlet (105) is provided with a fifth valve (110).

2. The device according to claim 1, wherein the device is airtight; preferably, the gas storage device (103) is capable of containing or discharging gas; for example, the gas storage device (103) is variable in volume, such as an elastic or stretchable container (e.g., air bag), or a container with a piston (e.g., syringe); preferably, a third valve (108) is arranged between the second container (102) and the gas storage device (103).

3. The device according to claim 1 or 2, wherein the first container (101) is tubular, one end of which is provided with a first valve (106) for connecting to a driving device (111), and the other end is provided with a second valve (107) for connecting to the second container (102);
preferably, the first container (101) has feature (3-1) or feature (3-2) as follows:
(3-1) the first container (101) contains a lysing solution for nucleic acid extraction (e.g., a cell lysing solution), a washing solution for nucleic acid extraction (e.g., a nucleic acid washing solution) and an eluting solution (e.g., a nucleic acid eluting solution) for nucleic acid extraction;
preferably, the lysing solution, the washing solution and the eluting solution are separated from each other;
preferably, the lysing solution, the washing solution and the eluting solution are arranged in the first container (101) from the proximal end to the distal end according to the distance from the second valve (107);
preferably, the lysing solution and the washing solution are separated by a first spacer;
preferably, the washing solution and the eluting solution are separated by a second spacer;
preferably, the first container (101) contains the lysing solution, the first spacer, the washing solution, the second spacer and the eluting solution, and they are arranged in turn in the first container (101) from the proximal end to the distal end according to the distance from the second valve (107);
preferably, the first spacer and the second spacer are the same or different;
preferably, the first spacer and the second spacer are each independently selected from the group consisting of air, mineral oil, animal oil, vegetable oil, synthetic oil, paraffin, silicone oil, and any combination thereof;
preferably, the washing solution comprises one or more portions, and the portions are separated from each other by an additional spacer or additional spacers; preferably, the portions have the same or different components; preferably, the additional spacers are each independently selected from the group consisting of air, mineral oil, animal oil, vegetable oil, synthetic oil, paraffin, silicone oil, and any combination thereof;
preferably, a third spacer is provided between the eluting solution and the first valve (106); preferably, the third spacer is selected from the group consisting of air, mineral oil, animal oil, vegetable oil, synthetic oil, paraffin, silicone oil, and any combination thereof;
or,
(3-2) the first container (101) contains a washing solution (e.g., a nucleic acid washing solution) and an eluting solution (e.g., a nucleic acid eluting solution) for nucleic acid extraction;
preferably, the washing solution and the eluting solution are separated from each other;
preferably, the washing solution and the eluting solution are arranged in the first container (101) from the proximal end to the distal end according to the distance from the second valve (107);
preferably, the washing solution and the eluting solution are separated by a first spacer;
preferably, the first container (101) contains the washing solution, the first spacer and the eluting solution, and they are arranged in turn in the first container (101) from the proximal end to the distal end according to the distance from the second valve (107);
preferably, the washing solution comprises one or more portions, and the portions are separated from each other by an additional spacer or additional spacers; preferably, the portions have the same or different components; preferably, the additional spacers are each independently selected from the group consisting of air, mineral oil, animal oil, vegetable oil, synthetic oil, paraffin, silicone oil, and any combination thereof;
preferably, a second spacer is provided between the eluting solution and the first valve (106);
preferably, the first spacer and the second spacer are the same or different;
preferably, the first spacer and the second spacer are each independently selected from the group consisting of air, mineral oil, animal oil, vegetable oil, synthetic oil, paraffin, silicone oil, and any combination thereof;
preferably, the second container (102) contains a lysing solution (e.g., a cell lysing solution) for nucleic acid extraction.

4. The device according to claim 1 or 2, wherein the first container (101) has a first chamber, a second chamber and a third chamber separated from each other; wherein the first chamber is used to accommodate or contain a lysing solution (e.g., a cell lysing solution) for nucleic acid extraction; the second chamber is used to accommodate or contain a washing solution (e.g., a nucleic acid washing solution) for nucleic acid extraction; the third chamber is used to accommodate or contain an eluting solution (e.g., a nucleic acid eluting solution) for nucleic acid extraction; and, the second valve (107) can control the type of reagent flowing into the second container (102); or,
the first container (101) has a first chamber and a second chamber separated from each other; wherein, the first chamber is used to accommodate or contain a washing solution (e.g., a nucleic acid washing solution) for nucleic acid extraction; the second chamber is used to accommodate or contain an eluting solution (e.g., a nucleic acid eluting solution) for nucleic acid extraction; and, the second valve (107) can control the type of reagent flowing into the second container (102); preferably, the second container (102) contains a lysing solution (e.g., a cell lysing solution) for nucleic acid extraction.

5. The device according to any one of claims 1-4, wherein the device has one or more technical features selected from the group consisting of the following:
(1) the first valve (106) is used to control the fluid communication between the first container (101) and the driving device (111); preferably, the device also has a driving device (111), which is in fluid communication with the first container (101) through the first valve (106); preferably, the driving device (111) is detachable; preferably, the driving device (111) is a driving pump;
(2) the second container (102) contains a magnet (e.g., a magnetic bead) capable of adsorbing a nucleic acid, and/or, a lysing solution (e.g., a cell lysing solution) for nucleic acid extraction; preferably, the magnetic bead is one or more selected from the following: silica gel plasma membrane magnetic bead, amino magnetic bead, hydroxyl magnetic bead, formyl magnetic bead, cellulose-coated magnetic bead;
(3) the sample outlet (105) is connected via the fifth valve (110) to the waste liquid pool (112), or the collection pool (113), or the waste liquid pool (112) and the collection pool (113); preferably, the fifth valve (110) is capable of controlling the flow direction of the fluid flowing through the fifth valve (110); preferably, the collection pool (113) contains a reagent for detecting the nucleic acid; preferably, the waste liquid pool (112) and/or the collection pool (113) are detachable;
(4) the nucleic acid is selected from the group consisting of DNA, RNA or any combination thereof.

6. A kit, which comprises the device according to any one of claims 1 to 5; preferably, the kit further comprises one or more parts or components selected from the group consisting of the following:
(6-1) a reagent for nucleic acid extraction; preferably, the reagent for nucleic acid extraction is selected from the group consisting of lysing solution (e.g., cell lysing solution), washing solution (e.g., nucleic acid washing solution), eluting solution (e.g., nucleic acid eluting solution), or any combination thereof; preferably, the lysing solution, the washing solution and the eluting solution are separated from each other;
(6-2) one or more spacers; preferably, the one or more spacers are selected from the group consisting of air, mineral oil, animal oil, vegetable oil, synthetic oil, paraffin, silicone oil, and any combination thereof;
(6-3) a magnet (e.g., a magnetic bead) capable of adsorbing a nucleic acid; preferably, the magnetic bead is one or more selected from the group consisting of the following: silica gel plasma membrane magnetic bead, amino magnetic bead, hydroxyl magnetic bead, formyl magnetic bead, cellulose-coated magnetic bead;
(6-4) a driving device; preferably, the driving device is a driving pump; preferably, the driving device can be in fluid communication with the first container (101) through the first valve (106);
(6-5) a waste liquid pool; preferably, the waste liquid pool can be in fluid communication with the sample outlet (105) through a fifth valve (110); and
(6-6) a collection pool; preferably, the collection pool can be in fluid communication with the sample outlet (105) through a fifth valve (110); preferably, the collection pool contains a reagent for detecting a nucleic acid.

7. A method for nucleic acid extraction, the method comprising, using the device according to any one of claims 1 to 5 or the kit according to claim 6 to extract a nucleic acid from a sample.

8. A method for nucleic acid extraction, the method comprising:
(1) providing a sample containing a nucleic acid, and the device according to any one of claims 1 to 5 or the kit according to claim 6;
(2) loading the sample into the second container (102) through the sample inlet (104), and closing the fourth valve (109); wherein, the second container (102) contains a magnet (e.g., a magnetic bead) capable of adsorbing the nucleic acid; preferably, the magnet has been preloaded in the second container (102), or, the magnet is loaded into the second container (102) together with the sample, or, the magnet is loaded into the second container (102) after the sample is loaded;
(3) contacting, mixing and incubating the lysing solution, the magnet (e.g., magnetic bead) capable of adsorbing the nucleic acid and the sample in the second container (102) for an appropriate time; preferably, the lysing solution has been pre-loaded into the second container (102), or the lysing solution is loaded into the second container (102) together with the sample, or the lysing solution is loaded into the second container (102) after the sample is loaded, or, the lysing solution has been loaded in the first container (101) in advance, and is driven into the second container (102) by the driving device (111), wherein, during the driving process, the first valve (106), the second valve (107) and the third valve (108) are in an open state, and the fourth valve (109) and the fifth valve (110) are in a closed state; after the driving process is completed, the first valve (106) and the second valve (107) are closed, and optionally the third valve (108) was closed;
(4) after incubation, opening the third valve (108) and the fifth valve (110), discharging all the solution in the second container (102) to the waste liquid pool (112) through the fifth valve (110), and retaining the magnet;
(5) closing the fifth valve (110), using the driving device (111) to drive the washing solution preloaded in the first container (101) into the second container (102), allowing it to contact, mix and incubate with the magnet for a suitable time; wherein, during the driving process, the first valve (106), the second valve (107) and the third valve (108) are in an open state, and the fourth valve (109) and the fifth valve (110) are in a closed state; after the driving process is completed, the first valve (106) and the second valve (107) are closed, and optionally the third valve (108) is closed;
(6) after incubation, opening the third valve (108) and the fifth valve (110), discharging all the solution in the second container (102) to the waste liquid pool (112) through the fifth valve (110), and retaining the magnet;
optionally, repeating steps (5) and (6) one or more times;
(7) closing the fifth valve (110), using the driving device (111) to drive the eluting solution preloaded in the first container (101) into the second container (102), allowing it to contact, mix and incubate with the magnet for a suitable time; wherein, in the driving process, the first valve (106), the second valve (107) and the third valve (108) are in an open state, and the fourth valve (109) and the fifth valve (110) are in a closed state; after the driving process is completed, the first valve (106) and the second valve (107) are closed, and optionally the third valve (108) is closed;
(8) after incubation, opening the third valve (108) and the fifth valve (110), and collecting all the solution in the second container (102) into the collection pool (113) through the fifth valve (110), thereby obtaining a solution containing the nucleic acid to be extracted.

9. Use of the device according to any one of claims 1 to 5 or the kit according to claim 6 for extracting a nucleic acid.
